(19) Europäisches Patentamt
European Patent Office
Office européen des brevets

(11) Veröffentlichungsnummer: **0 392 298 A2**

# (12) EUROPÄISCHE PATENTANMELDUNG

(21) Anmeldenummer: **90106215.8**

(22) Anmeldetag: **31.03.90**

(51) Int. Cl.⁵: **C07D 239/54, C07C 275/50, A01N 43/54**

(30) Priorität: **13.04.89 DE 3912100**

(43) Veröffentlichungstag der Anmeldung:
**17.10.90 Patentblatt 90/42**

(84) Benannte Vertragsstaaten:
**AT BE CH DE DK ES FR GB IT LI NL**

(71) Anmelder: **BAYER AG**
**D-5090 Leverkusen 1 Bayerwerk(DE)**

(72) Erfinder: **Lindner, Werner, Dr.**
**Märchenstrasse 39**
**D-5000 Köln 80(DE)**
Erfinder: **Haberkorn, Axel, Prof. Dr.**
**Fuhlrottstrasse 99**
**D-5600 Wuppertal 1(DE)**

## (54) Substituierte Uracile, Verfahren zu ihrer Herstellung und ihre Verwendung gegen parasitäre Protozoen.

(57) Die vorliegende Erfindung betrifft neue substituierte Uracile allgemeinen Formel (I)

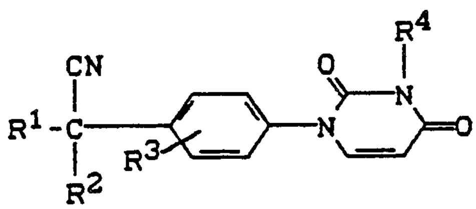

(I)

in welcher
$R^1$ für aromatische oder heteroaromatische Reste steht, die gegebenenfalls substituiert sind,
$R^2$ für H, Alkyl, Alkenyl, Alkinyl oder Aralkyl steht, die gegebenenfalls substituiert sind,
$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl,
$R^4$ für Wasserstoff, einen geradkettigen, verzweigten oder cyclischen Alkylrest, Alkenyl, Alkinyl oder Aralkyl steht, die gegebenenfalls substituiert sind,
Verfahren zu ihrer Herstellung und ihre Verwendung gegen parasitäre Protozoen.

EP 0 392 298 A2

## Substituierte Uracile, Verfahren zu ihrer Herstellung und ihre Verwendung gegen parasitäre Protozoen

Die vorliegende Erfindung betrifft neue substituierte Uracile, Verfahren zu ihrer Herstellung, Zwischenprodukte zur Durchführung dieser Verfahren sowie ihre Verwendung gegen parasitäre Protozoen.

Die Verwendung von F-substituiertem Uracil zur Bekämpfung von Coccidien ist bekannt. Die Wirkung dieser Verbindung befriedigt jedoch nicht in jedem Fall (US-P 3 017 322).

Die vorliegende Erfindung betrifft

1. Neue substituierte Uracile der allgemeinen Formel (I)

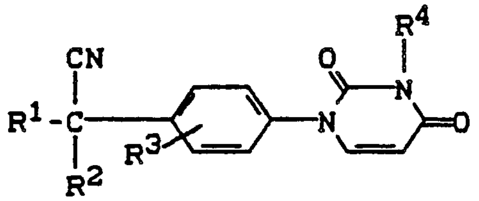

(I)

in welcher
$R^1$ für aromatische oder heteroaromatische Reste steht, die gegebenenfalls substituiert sind,
$R^2$ für H, Alkyl, Alkenyl, Alkinyl oder Aralkyl steht, die gegebenenfalls substituiert sind,
$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano Alkoxycarbonyl, Alkylsulfonyl, Halogenalkylsulfonyl,
$R^4$ für Wasserstoff, einen geradkettigen, verzweigten oder cyclischen Alkylrest, Alkenyl, Alkinyl oder Aralkyl steht, die gegebenenfalls substituiert sind.

2. Verfahren zur Herstellung substituierter Uracile der allgemeinen Formel (Ia)

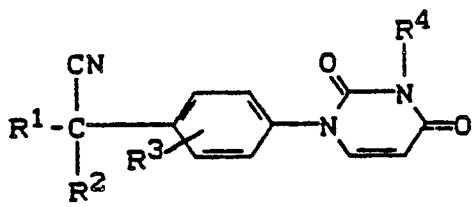

(I)

in welcher
$R^1$ für aromatische oder heteroaromatische Reste steht, die gegebenenfalls substituiert sind,
$R^2$ für Wasserstoff, Alkyl, Alkenyl, Alkinyl oder Aralkyl steht, die gegebenenfalls substituiert sind,
$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl,
$R^4$ für Wasserstoff, einen geradkettigen, verzweigten oder cyclischen Alkylrest, Alkenyl, Alkinyl oder Aralkyl steht, die gegebenenfalls substituiert sind,
indem man
a) Verbindungen der Formel (II)

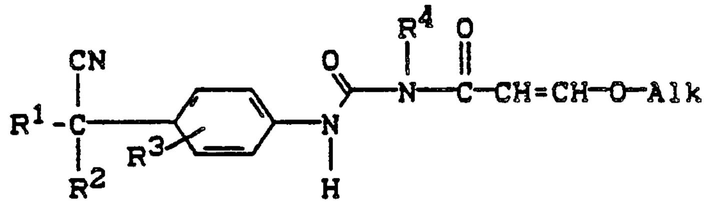

(II)

in welcher
$R^1$, $R^2$, $R^3$ und $R^4$ die oben angegebenen Bedeutungen haben und

Alk für $C_1$-$C_4$-Alkyl insbesondere Ethyl steht,
gegebenenfalls in Gegenwart von Basen erhitzt, oder indem man
b) Verbindungen der Formel (Ia)

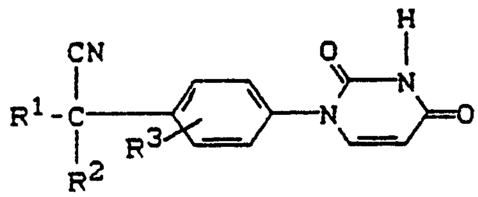

(Ia)

in welcher $R^1$, $R^2$, $R^3$ die oben angegebenen Bedeutungen haben,
mit Verbindungen der Formel (III)

$R^4$ - A

in welcher
$R^4$ für gegebenenfalls substituiertes Alkyl, Alkenyl, Alkinyl oder Aralkyl steht und
A für Halogen, -$OSO_2$-Alkyl, -$OSO_2$-Aryl, -$OSO_2$-Halogenalkyl steht,
umsetzt oder indem man
c) Verbindungen der Formel (IV)

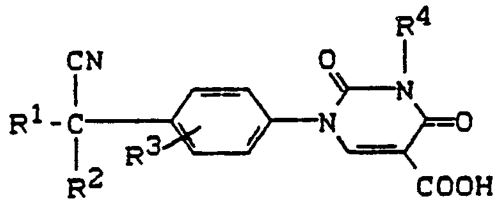

(IV)

in welcher
$R^1$, $R^2$, $R^3$ die oben angegebenen Bedeutungen haben,
durch Erhitzen decarboxyliert.

3. Neue Verbindungen der Formel (II)

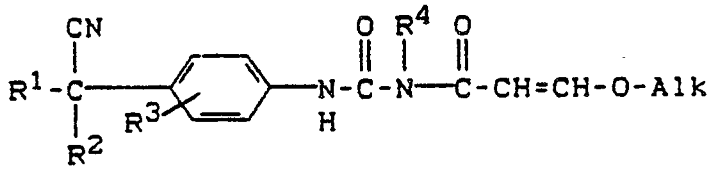

(II)

in welcher
$R^1$, $R^2$, $R^3$, $R^4$, Alk die bei Verfahren 2a) angegebenen Bedeutungen haben.

4. Verfahren zur Herstellung der Verbindungen der Formel (II) gemäß 3, dadurch gekennzeichnet, daß man
a) Verbindungen der Formel (V)

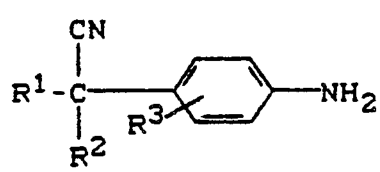

(V)

in welcher
$R^1$, $R^2$, $R^3$ die obengenannten Bedeutungen besitzen,
mit Isocyanaten der Formel (VI)

$$O=C=N-\overset{\overset{O}{\|}}{C}-CH=CH-O-Alk \quad (VI)$$

in welcher

Alk die obengenannte Bedeutung besitzt,

umsetzt, oder

b) Verbindungen der Formel (VII)

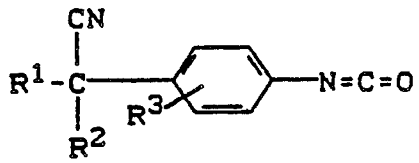

(VII)

in welcher $R^1$, $R^2$, $R^3$ die obengenannten Bedeutungen besitzen,

mit Verbindungen der Formel (VIII)

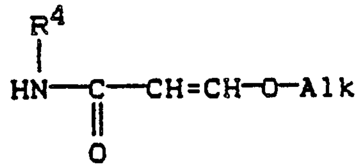

(VIII)

in welcher

$R^4$ oder Alk die obengenannten Bedeutungen besitzt, umsetzt.

5. Neue Verbindungen der Formel (IV)

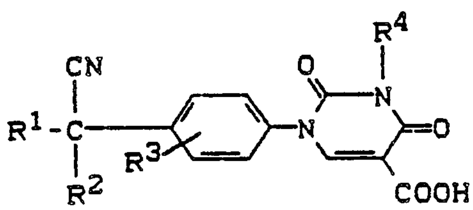

(IV)

in welcher

$R^1$, $R^2$, $R^3$, $R^4$ die oben angegebenen Bedeutungen besitzen.

6. Verfahren zur Herstellung der neuen Verbindungen der Formel (IV) gemäß 5, dadurch gekennzeichnet, daß man Verbindungen der Formel (IX)

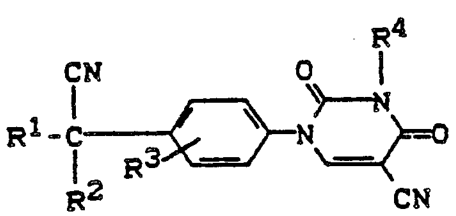

(IX)

in welcher

$R^1$, $R^2$, $R^3$ und $R^4$ die oben angegebenen Bedeutungen besitzen,

in Gegenwart wäßriger Säuren erhitzt.

7. Neue Verbindungen der Formel (IX)

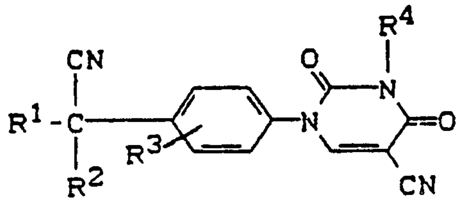

(IX)

in welcher
$R^1$, $R^2$, $R^3$, $R^4$ die oben angegebenen Bedeutungen haben.

8. Verfahren zur Herstellung der neuen Verbindungen der Formel (IX) gemäß 7., dadurch gekennzeichnet, daß man Verbindungen der Formel (X)

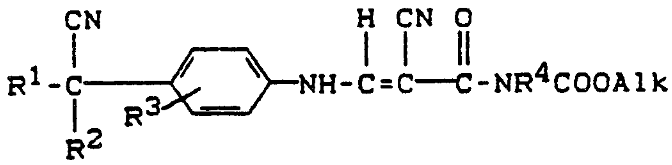

(X)

in welcher
$R^1$, $R^2$, $R^3$, $R^4$, Alk die oben angegebene Bedeutung besitzen,
in Gegenwart von Basen erhitzt.

9. Neue Verbindungen der Formel (X)

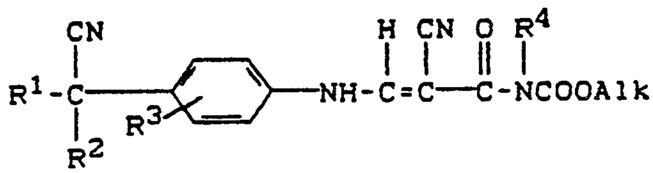

(X)

in welcher
$R^1$, $R^2$, $R^3$, $R^4$ und Alk die unter 4 b) beschriebenen Bedeutungen besitzen.

10. Verfahren zur Herstellung der neuen Verbindungen der Formel (X) gemäß 9., dadurch gekennzeichnet, daß man Verbindungen der Formel (V)

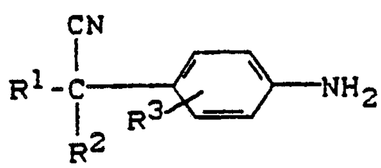

(V)

in welcher
$R^1$, $R^2$, $R^3$ die unter 4 b) beschriebenen Bedeutungen besitzen
mit Verbindungen der Formel (XI)

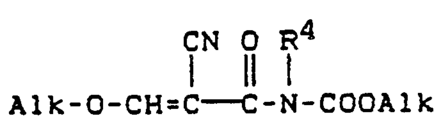

(XI)

in welcher
Alk für gleiches oder verschiedenes $C_1$-$C_4$-Alkyl steht,
umsetzt.

Die Verbindungen der Formel (I) sowie ihre Salze mit Säuren oder Basen sind hervorragend zur

Bekämpfung parasitischer Protozoen geeignet.

Bevorzugte Verbindungen der Formel (I) sind Verbindungen, in denen

$R^1$ für gegebenenfalls durch Halogen, Alkyl, Cyano, Alkoxy, Alkylthio, Methylendioxy, Ethylendioxy, Alkylsulfinyl, Alkylsulfonyl, Halogenalkyl, Halogenalkoxy, Halogenalkylthio, Halogenalkylsulfinyl, Halogenalkylsulfonyl substituiertes Thiazolyl, Oxazolyl, Benzthiazolyl, Benzoxazolyl, Pyridinyl, Pyrimidinyl, Pyrazinyl, Indolyl, Benzimidazolyl, Phenyl oder Naphthyl steht,

$R^2$ für H oder Alkyl steht,

$R^3$ für einen oder mehrere, gleiche oder verschiedene Reste der Gruppe Halogen, Alkyl, Halogenalkyl, Al koxy, Halogenalkoxy, Alkylthio, Halogenalkylthio oder Cyano steht,

$R^4$ für Wasserstoff, Alkyl, Alkenyl oder Alkinyl steht, die gegebenenfalls durch Halogen, Halogenalkyl, Alkoxy, Alkylthio, Aryloxy, Arylthio oder Aryl substituiert sind.

Besonders bevorzugt sind Verbindungen der Formel (I), in welcher

$R^1$ für Phenyl, Pyridyl, Benzthiazolyl steht, die durch einen oder mehrere gleiche oder verschiedene Reste der Gruppe Halogen, $C_{1-4}$-Alkyl, $C_{1-4}$-Halogenalkyl, $C_{1-4}$-Alkoxy, $C_{1-4}$-Alkylthio, $C_{1-4}$-Halogenalkoxy, $C_{1-4}$-Halogenalkylthio, Cyano, $C_{1-4}$-Alkoxycarbonyl, $C_{1-4}$-Alkylsulfinyl, $C_{1-4}$-Alkylsulfonyl, $C_{1-4}$-Halogenalkylsulfinyl, $C_{1-4}$-Halogenalkylsulfonyl substituiert ist,

$R^2$ für H oder $C_{1-4}$-Alkyl steht,

$R^3$ für einen oder mehrere gleiche oder verschiedene Reste der Gruppe Wasserstoff, Halogen, $C_{1-4}$-Alkyl, $C_{1-4}$-Halogenalkyl steht,

$R^4$ für Wasserstoff steht.

Ganz besonders bevorzugt sind Verbindungen der Formel (I), in welcher

$R^1$ für Phenyl steht, das gegebenenfalls durch Halogen, insbesondere Chlor, Brom, Fluor, $C_{1-4}$-Halogenalkyl, insbesondere Trifluormethyl, $C_{1-4}$-Halogenalkylthio, insbesondere Trifluormethylthio, $C_{1-4}$-Halogenalkoxy, insbesondere Trifluormethoxy, $C_{1-4}$-Alkyl, insbesondere Methyl, $C_1$-$C_4$- Halogenalkylsulfinyl wie Trifluormethylsulfinyl, $C_1$-$C_4$-Halogenalkylsulfonyl wie Trifluormethylsulfonyl substituiert ist,

$R^2$ für H oder $C_{1-4}$-Alkyl, insbesondere Methyl steht,

$R^3$ für Halogen, insbesondere Brom, Chlor, Fluor, $C_{1-4}$-Alkyl, insbesondere Methyl, Halogenalkyl, insbesondere Trifluormethyl steht,

$R^4$ für Wasserstoff steht.

Als Einzelverbindungen seien genannt:

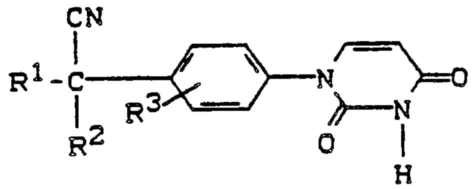

| $R^1$ | $R^2$ | $R^3$ |
|---|---|---|
| 4-Cl-Phenyl | H | 3,5 $Cl_2$ |
| 4-$SCF_3$-Phenyl | H | 3,5 $Cl_2$ |
| 4-$OCF_3$-Phenyl | H | 3,5 $Cl_2$ |
| 4-$SOCF_3$-Phenyl | H | 3,5 $Cl_2$ |
| 4-$SO_2$ $CF_3$-Phenyl | H | 3,5 $Cl_2$ |
| 4-$CF_3$-Phenyl | H | 3,5 $Cl_2$ |
| 3-Cl-4$CF_3$-Phenyl | H | 3,5 $Cl_2$ |
| 3,4-$Cl_2$-Phenyl | H | 3,5 $Cl_2$ |

Weiterhin seien die folgenden Verbindungen genannt:

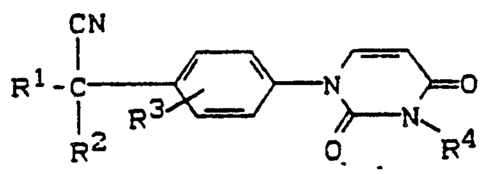

| $R^1$ | $R^2$ | $R^3$ | $R^4$ |
|---|---|---|---|
| 2-Benzthiazolyl | H | 3-Cl | H |
| 2-Benzthiazolyl | H | 3,5-$Cl_2$ | H |
| 2-(6-Cl-Benzthiazolyl) | H | 3-Cl | H |
| 2-(6-Cl-Benzthiazolyl) | H | 3,5-$Cl_2$ | H |
| 2-(5,6-Cl-Benzthiazolyl) | H | 3-Cl | H |
| 2-(5,6-Cl-Benzthiazolyl) | H | 3,5-$Cl_2$ | H |
| 2-Benzoxazolyl | H | 3-Cl | H |
| 2-Benzoxazolyl | H | 3,5-$Cl_2$ | H |
| 2-Pyridinyl | H | 3-Cl | H |
| 3-Pyridinyl | H | 3,5-$Cl_2$ | H |

| $R^1$ | $R^2$ | $R^3$ | $R^4$ |
|---|---|---|---|
| 4-Cl Phenyl | H | 3-$CH_3$ | H |
| 4-Cl Phenyl | H | 3-Cl | H |
| 4-Cl Phenyl | H | 3-Cl,5-$CH_3$ | H |
| 4-Cl Phenyl | H | 3,5-$Br_2$ | H |
| 4-Cl Phenyl | H | 3-$CF_3$ | H |
| 4-$CF_3$ Phenyl | H | 3-$CH_3$ | H |
| 4-$CF_3$ Phenyl | H | 3-Cl | H |
| 4-$CF_3$ Phenyl | H | 3-Cl,5-$CH_3$ | H |
| 4-$CF_3$ Phenyl | H | 3,5-$Br_2$ | H |
| 4-$CF_3$ Phenyl | H | 3-$CF_3$ | H |
| 4-F-Phenyl | H | 3,5-$Cl_2$ | H |
| 3,4-$Cl_2$-Phenyl | H | 3-$CH_3$ | H |
| 3,4-$Cl_2$-Phenyl | H | 3-Cl | H |
| 3,4-$Cl_2$-Phenyl | H | 3-Cl,5-$CH_3$ | H |
| 3,4-$Cl_2$-Phenyl | H | 3,5-$Br_2$ | H |
| 3,4-$Cl_2$-Phenyl | H | 3-$CF_3$ | H |
| 3,4-$Cl_2$-Phenyl | H | 3-$CH_3$ | H |
| 2,4-$Cl_2$-Phenyl | H | 3-$CH_3$ | H |
| 2,4-$Cl_2$-Phenyl | H | 3-Cl | H |
| 2,4-$Cl_2$-Phenyl | H | 3-Cl,5-$CH_3$ | H |
| 2,4-$Cl_2$-Phenyl | H | 3,5-$Br_2$ | H |
| 2,4-$Cl_2$-Phenyl | H | 3-$CF_3$ | H |
| 3-Cl-Phenyl | H | 3-$CH_3$ | H |
| 3-Cl-Phenyl | H | 3-Cl | H |

| $R^1$ | $R^2$ | $R^3$ | $R^4$ |
|---|---|---|---|
| 3-Cl-Phenyl | H | 3-Cl,5-$CH_3$ | H |
| 3-Cl-Phenyl | H | 3,5-$Br_2$ | H |
| 3-Cl-Phenyl | H | 3-$CF_3$ | H |
| 4-$SCF_3$-Phenyl | H | 3-$CH_3$ | H |
| 4-$SCF_3$-Phenyl | H | 3-Cl | H |
| 4-$SCF_3$-Phenyl | H | 3-Cl,5-$CH_3$ | H |
| 4-$SCF_3$-Phenyl | H | 3,5-Br | H |
| 4-$SCF_3$-Phenyl | H | 3-$CF_3$ | H |
| 4-$SCF_3$-Phenyl | H | 3-$CH_3$ | H |
| 4-$OCF_3$-Phenyl | H | 3-$CH_3$ | H |
| 4-$SCF_3$-Phenyl | $CH_3$ | 3,5-$Cl_2$ | H |
| 4-$OCF_3$-Phenyl | $CH_3$ | 3,5-$Cl_2$ | H |
| 4-$OCF_3$-Phenyl | H | 3-Cl | H |
| 4-$OCF_3$-Phenyl | H | 3-Cl,5-$CH_3$ | H |
| 4-$OCF_3$-Phenyl | H | 3,5-$Br_2$ | H |
| 4-$OCF_3$-Phenyl | H | 3-$CF_3$ | H |
| 4-CN-Phenyl | H | 3-$CH_3$ | H |
| 4-CN-Phenyl | H | 3-Cl | H |
| 4-CN-Phenyl | H | 3-Cl,5-$CH_3$ | H |
| 4-CN-Phenyl | H | 3,5-$Br_2$ | H |
| 4-CN-Phenyl | H | 3-$CF_3$ | H |
| 3-$OCH_3$-Phenyl | H | 3-$CH_3$ | H |

| $R^1$ | $R^2$ | $R^3$ | $R^4$ |
|---|---|---|---|
| 4-$OCH_3$-Phenyl | H | 3-Cl | H |
| 4-$OCH_3$-Phenyl | H | 3-Cl,5-$CH_3$ | H |
| 4-$OCH_3$-Phenyl | H | 3,5-$Br_2$ | H |
| 4-$OCH_3$-Phenyl | H | 3-$CF_3$ | H |
| 4-$SO_2CF_3$-Phenyl | H | 3-$CH_3$ | H |
| 4-$SO_2CF_3$-Phenyl | H | 3-Cl | H |
| 4-$SO_2CF_3$-Phenyl | H | 3-Cl,5-$CH_3$ | H |
| 4-$SO_2CF_3$-Phenyl | H | 3,5-$Br_2$ | H |
| 4-$SO_2CF_3$-Phenyl | H | 3-$CF_3$ | H |
| 4-$SOCF_3$-Phenyl | H | 3-$CH_3$ | H |
| 4-$SOCF_3$-Phenyl | H | 3-Cl | H |
| 4-$SOCF_3$-Phenyl | H | 3-Cl,5-$CH_3$ | H |
| 4-$SOCF_3$-Phenyl | H | 3,5-$Br_2$ | H |
| 4-$SOCF_3$-Phenyl | H | 3-$CF_3$ | H |
| 2-Benzimidazolyl | H | 3-Cl | H |
| 2-Indolyl | H | 3-Cl | H |

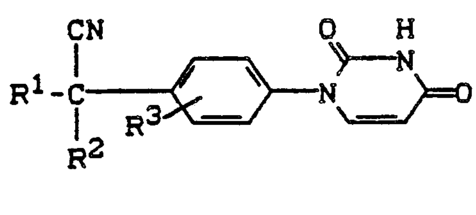

| $R^1$ | $R^2$ | $R^3$ | $R^4$ |
|---|---|---|---|
| 2-Thienyl | H | 3,5-$Cl_2$ | H |
| 4-$SCF_3$-Phenyl | $CH_3$ | 3,5-$Cl_2$ | $CH_3$ |
| 4-Cl-Phenyl | $CH_3$ | 3,5-$Cl_2$ | $CH_3$ |

Wird im Verfahren 2a) als Verbindung der Formel (II) 2,6-Dichlor-α-(4′-chlorphenyl)-4-(3-ethoxyacryloyl)-ureido-phenylacetonitril eingesetzt, läßt sich das Verfahren durch folgendes Schema beschreiben:

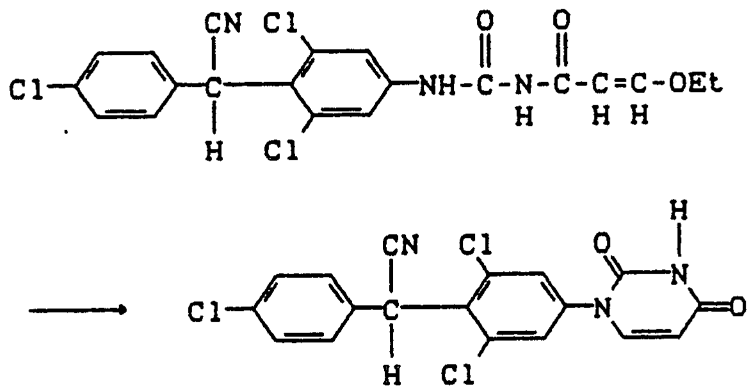

Die Verbindungen der Formel (II) sind neu. Sie sind nach dem unter 4 beschriebenen Verfahren erhältlich.

Bevorzugt seien Verbindungen der Formel (II) genannt, in den $R^1$, $R^2$, $R^3$ die bei den Verbindungen der Formel (I) genannten bevorzugten Bedeutungen haben.

Im einzelnen seien folgende Verbindungen der Formel (II) genannt:

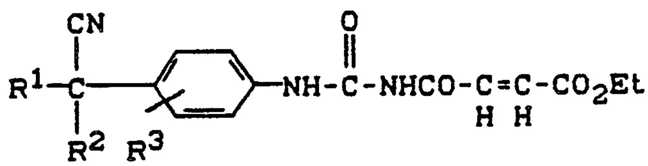

| $R^1$ | $R^2$ | $R^3$ |
|---|---|---|
| 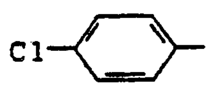 | H | 3,5-$Cl_2$ |
| 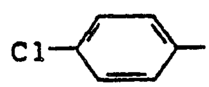 | H | 3-$Cl_2$ |
| 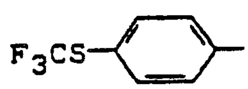 | H | 3,5-$Cl_2$ |
| 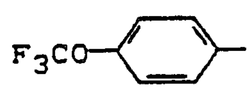 | H | 3,5-$Cl_2$ |
| 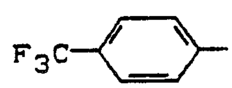 | H | 3,5-$Cl_2$ |
| 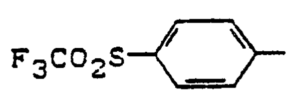 | H | 3,5-$Cl_2$ |

Das Verfahren 2a) wird durchgeführt, indem man eine Verbindung der Formel (II) in Substanz oder in Lösung, gegebenenfalls in Form ihres Salzes erhitzt. Nach abgeschlossener Umsetzung wird das Reaktionsgemisch mit verdünnter anorganischer Säure (z.B. Salzsäure) angesäuert und der ausgefallene Feststoff abfiltriert.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin,

Ligroin, Benzol, Toluol, Xylol, Methylenchlorid, Ethylenchlorid, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-, Ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid, Alkohole wie Ethanol oder tert. Butanol.

Die Reaktion wird in Gegenwart von anorganischen oder organischen Basen durchgeführt.

Als solche seien genannt z.B.:

Alkalimetallhydroxide wie z.B. Natrium- und Kaliumhydroxid, Erdalkalihydroxide wie z.B. Calciumhydroxid, Alkaliacetate wie Natriumacetat, Alkalicarbonate und -alkoholate wie Natrium- und Kaliumcarbonat, Natrium- und Kaliummethylat bzw. -ethylat oder Kalium tert. Butylat, ferner aliphatische, aromatische oder heterocyclische Amine, beispielsweise Triethylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Umsetzung erfolgt bei Temperaturen zwischen 80 °C und 200 °C, vorzugsweise zwischen 80 °C und 160 °C, bei Normaldruck oder erhöhtem Druck. Es wird bevorzugt bei Normaldruck gearbeitet.

Setzt man bei dem Verfahren 2b) als Verbindungen der Formel (Ia) 2,6-Dichlor-α-(2′,4′-dichlorphenyl)-α-methyl-4-(1-uracil)-phenylacetonitril und als Verbindung der Formel (III) Methyliodid ein, kann das Verfahren durch folgendes Formelschema beschrieben werden:

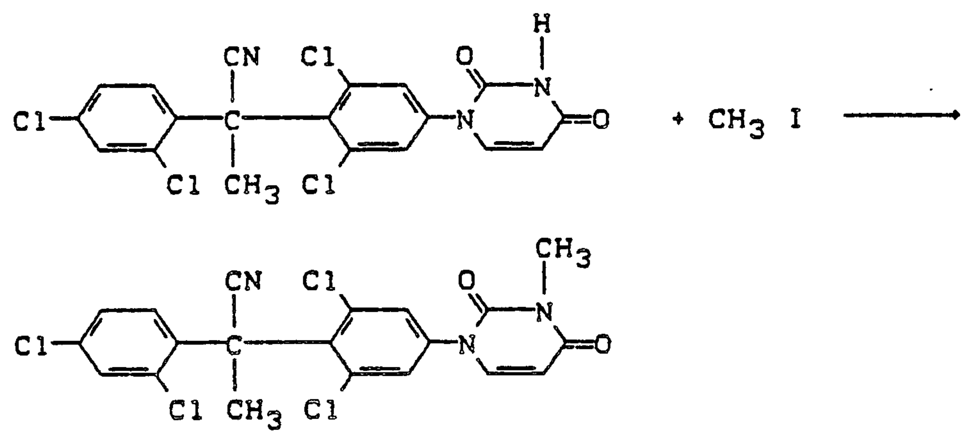

Die Verbindungen der Formel (Ia) sind neu und werden wie bei Verfahren 2a) beschrieben dargestellt.

Die Verbindungen der Formel (IV) sind bekannt oder lassen sich nach bekannten Methoden darstellen. Besonders genannt sei Methyliodid, Ethylbromid.

Das Verfahren wird durchgeführt, indem man eine Verbindung der Formel (Ia) in Gegewart einer Base und eines Verdünnungsmittels mit Verbindungen der Formel (IV) umsetzt. Als Verdünnungsmittel können alle inerten organischen Lösungsmittel eingesetzt werden, die auch zur Durchführung von Verfahren (2a) dienen.

Das Verfahren wird in Gegenwart von Basen durchgeführt. Als bevorzugte Basen seien genannt, die Alkalihydroxide wie Natriumhydroxid, Alkalialkoholate wie Natriummethylat oder Kaliumbutanolat, Metallhydride wie Natriumhydrid oder organische Basen wie 1,8-Diazabicyclo[5-undec-7-en (DBU).

Das Verfahren wird durchgeführt bei Normaldruck und Tempraturen zwischen 20 °C und 140 °C.

Die Reaktion wird durchgeführt, indem man äquimolare Mengen der Verbindung der Formel (Ia) und Base zusammengibt, dieses Gemisch mit einer äquimolaren Menge der Verbindung der Formel (IV) versetzt und auf die Reaktionstemperatur erhitzt.

Setzt man bei dem Verfahren 2c) zur Herstellung der Verbindungen (I) als Verbindung der Formel (IV) 2,6-Dichlor-α-(2′,6′-dichlorphenyl)-4-(5-carboxy-1-uracil)-phenylacetonitril ein, läßt sich das Verfahren durch folgendes Formelschema beschreiben:

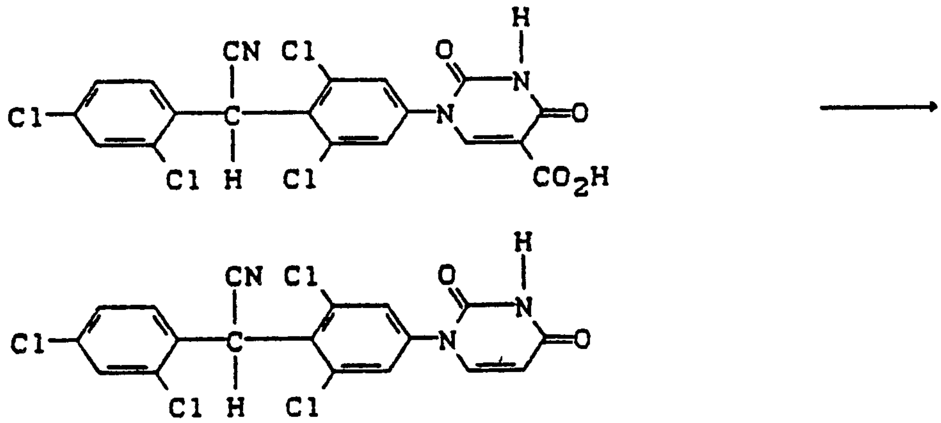

Die Verbindungen der Formel (IV) sind neu. Ihre Herstellung erfolgt nach dem unter (6) beschriebenen Verfahren.

Bevorzugt werden Verbindungen der Formel (IV) eingesetzt, in der $R^1$, $R^2$, $R^3$ die bei den Verbindungen der Formel (I) genannten bevorzugten und besonders bevorzugten Bedeutungen besitzt.

Die Decarboxylierung wird durch Erhitzen in Gegenwart von mercaptogruppenhaltigen Carbonsäuren wie z.B. Mercaptoessigsäure oder Thiosalicylsäure, gegebenenfalls in Gegenwart von inerten organischen Verdünnungsmitteln durchgeführt. Zu den Verdünnungsmitteln gehören aliphatische und aromatische, gegebenenfalls halogenierte Kohlenwasserstoffe wie Nonan, Decan, Dodecan, Xylole, Ether wie Ethylenglykolmonobutylether, Diethylenglykoldibutylether.

Die Umsetzung erfolgt bei Temperaturen zwischen 150°C und 300°C, vorzugsweise zwischen 160°C und 250°C, insbesondere zwischen 170°C und 210°C.

Es wird bei Normaldruck gearbeitet. Die Verbindungen der Formel (IV) werden in Substanz oder im jeweiligen Verdünnungsmittel, gelöst oder suspendiert, zusammen mit der mercaptogruppenhaltigen Carbonsäure erhitzt.

Wird im Verfahren 4 zur Herstellung der Verbindungen der Formel (II) als Verbindung der Formel (V) 2,6-Dichlor-α-(4′-chlorphenyl)-4-aminophenylacetonitril eingesetzt und als Verbindung der Formel (VI) Ethoxyacryloylisocyanat eingesetzt läßt sich das Verfahren durch folgendes Schema beschreiben:

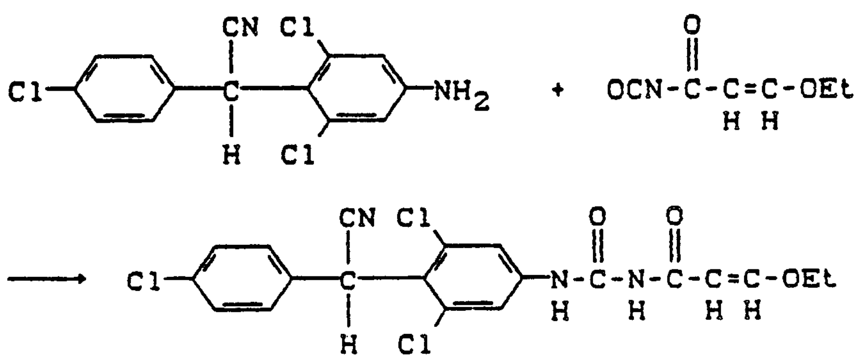

Die Verbindungen der Formel (V) sind zum Teil bekannt (US-P 4 005 218) oder können nach den dort beschriebenen Verfahren hergestellt werden. Zum Teil sind sie Gegenstand einer noch nicht veröffentlichten Anmeldung der Anmelderin (DE-Anm 3 834 272.3)

Bevorzugt seien Verbindungen der Formel (V) genannt, in der $R^1$, $R^2$, $R^3$ die bei den Verbindungen der Formel (I) genannten, bevorzugten oder besonders bevorzugten Bedeutungen besitzen. Im einzelnen seien die Verbindungen der folgenden Formel genannt:

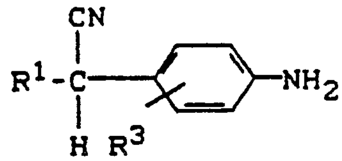

| $R^1$ | $R^3$ |
|---|---|
| 2-(6-Chlorbenzthiazolyl) | 3,5-$Cl_2$ |
| 2-Benzthiazolyl | 3-Cl |

| $R^1$ | $R^3$ |
|---|---|
| 2-Benzthiazolyl | 3-$CH_3$ |
| 2-Benzthiazolyl | 3,5-$Cl_2$ |
| 2-Benzimidazolyl | 3-Cl |
| 2-Indolyl | 3-Cl |
| 2-Pyridinyl | 3,5-$Cl_2$ |
| 3-Pyridinyl | 3,5-$Cl_2$ |
| 4-Cl-Phenyl | 3-Cl |
| 4-Cl-Phenyl | 3,5-$Cl_2$ |
| 3,4-Dichlorphenyl | 3,5-$Cl_2$ |
| $H_3CSO_2$-Phenyl | 3,5-$Cl_2$ |

Die Verbindungen der Formel (VI) sind bekannt.

Die Reaktion wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutyl-keton, Ester wie Essigsäuremethylester und -ethylester, Nitrile wie z. B. Acetonitril und Propionitril, Dimethylacetamid und N-Methyl-pyrrolidon sowie Dimethylsulfoxid, Tetramethylensulfon und Hexamethylphosphorsäuretriamid.

Die Umsetzung erfolgt bei Temperaturen zwischen 20°C und 180°C, vorzugsweise zwischen 30°C und 50°C.

Das Verfahren wird durchgeführt, indem etwa äquimolare Mengen der Verbindungen der Formel (V) und (VI) in einem der angegebenen Verdünnungsmittel zusammengegeben und erhitzt werden. Nach vollendeter Umsetzung wird abgekühlt und der ausgefallene Feststoff abfiltriert, gewaschen und getrocknet.

Setzt man bei dem Verfahren 4b) zur Herstellung der Verbindungen der Formel (II) als Verbindung der Formel (VII) 4-Isocyanato-2,6-dichloro-α-(4-chlorphenyl)-α-methyl-phenylacetonitril ein und als Verbindung der Formel (VIII) Ethoxyacryloyl-N-methylamid ein, läßt sich das Verfahren durch folgendes Formelschema beschreiben:

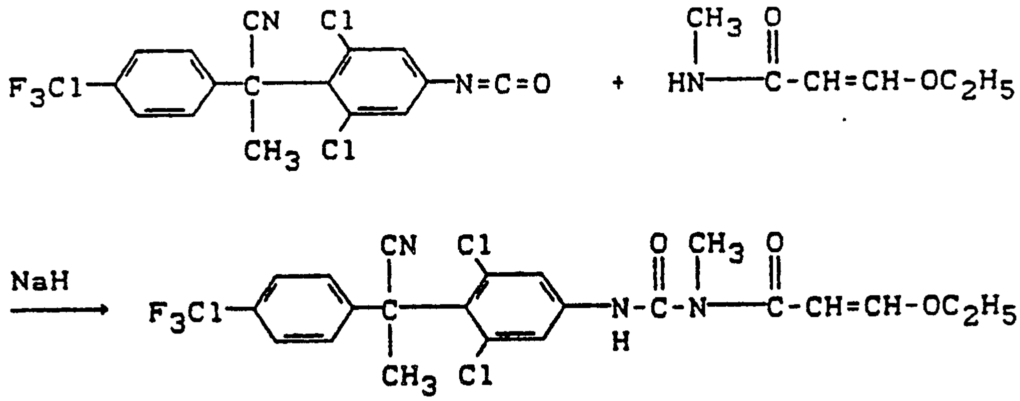


Als Einzelverbindungen der Formel (VII) seien genannt:

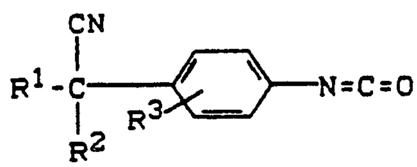

| $R^1$ | $R^2$ | $R^3$ |
|---|---|---|
| Benzthiazolyl | H | 3,5-Cl |
| 4-Cl Ph | H | 3-Cl |
| 4-Cl Ph | H | 3,5-Cl |
| 4-$OCF_3$ Ph | H | 3-$CH_3$ |
| 4-$OCF_3$ Ph | H | 3,5-Cl |

Das Verfahren wird durchgeführt, indem man etwa äquimolare Mengen der Verbindung der Formel (VII) und Verbindung der Formel (VIII) gegebenenfalls in Gegenwart eines Verdünnungsmittels und einer Base erhitzt. Als Verdünnungsmittel finden die bei der Herstellung der Verbindungen (I) aufgeführten Lösungsmittel Verwendung. Zusätzlich genannt sei Pyridin. Als Basen finden die bei Verfahren 2b beschrieben Verwendung. Als besonders bevorzugt sei Natriumhydrid genannt.

Die Umsetzung erfolgt unter Normal- oder erhöhtem Druck bei Temperaturen zwischen 20°C und 150°C, vorzugsweise zwischen 30°C und 80°C.

Die Verbindungen werden bevorzugt in äquimolaren Verhältnissen eingesetzt und das nach abgeschlossener Reaktion als Feststoff anfallende Produkt abfiltriert.

Wird im Verfahren 6) zur Herstellung der Verbindung (IV) als Verbindung der Formel (IX) 2,6-Dichlor-α-(4′-methylphenyl )-4-(5-cyano-1-uracil)phenylacetonitril eingesetzt, läßt sich das Verfahren durch folgendes Schema beschreiben:

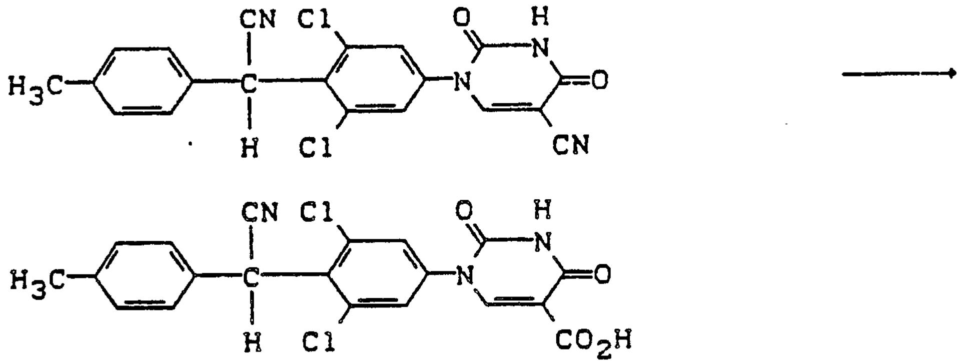

Die Verbindungen der Formel (IX) sind neu. Ihre Herstellung erfolgt nach dem unter 8) beschriebenen Verfahren. Im einzelnen seien folgende Verbindungen der Formel (IX) genannt:

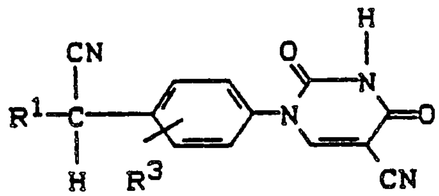

| $R^1$ | $R^3$ |
|---|---|
| 4-Cl-Phenyl | 3,5-$Cl_2$ |
| 4-Cl-Phenyl | 3-Cl |
| 4-$CF_3$-Phenyl | 3,5-$Cl_2$ |
| 4-$SO_2CF_3$-Phenyl | 3,5-$Cl_2$ |
| 4-$CF_3$-Phenyl | 3,5-$Cl_2$ |
| 4-$OCF_3$-Phenyl | 3,5-$Cl_2$ |
| 2-Pyridyl | 3,5-$Cl_2$ |
| 3,4-Cl-Phenyl | 3,5-$Cl_2$ |
| 4-$SCF_3$-Phenyl | 3-Cl |
| 4-$OCF_3$-Phenyl | 3-Cl |
| 4-$CH_3$-Phenyl | 3-Cl |
| 2-Benzthiazolyl | 3,5-$Cl_2$ |
| 4-$SO_2CH_3$-Phenyl | 3,5-Cl |

Die Hydrolyse wird unter sauren Bedingungen durchgeführt. Als Säuren kommen Mineralsäuren zum Einsatz, wie z.B. Salzsäure, Bromwasserstoffsäure, Schwefelsäure und Gemische aus Mineralsäuren und organischen Säuren, wie z.B. Essigsäure oder Propionsäure.

Die Umsetzung erfolgt bei Temperaturen zwischen 80 °C und 120 °C. Es wird unter Normaldruck gearbeitet.

Die Verbindungen der Formel (IX) werden im 10- bis 30-fachen Volumen der Säure oder des Säuregemisches gelöst und bis zur abgechlossenen Hydrolyse erhitzt.

In einer Variante können ausgehend von den Verbindungen der Formel (IX) direkt die Verbindungen der

Formel (I) erhalten werden. Dies wird erreicht durch Zusatz von bei Verfahren 2c) beschriebenen mercaptogruppenhaltigen Carbonsäuren, vorzugsweise Mercaptoessigsäure zum Hydrolysemedium.

Wird im Verfahren 8) zur Herstellung der Verbindungen (IX) als Verbindung der Formel (X) 2,6-Dichloro-α-(4′-methylphenyl)-4-N-(2-Cyano-acryloylurethan)amino-phenylacetonitril eingesetzt, läßt sich das Verfahren durch folgendes Schema beschreiben:

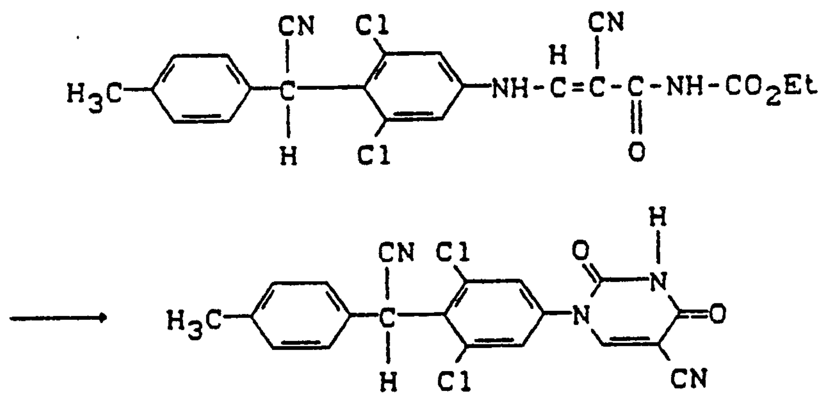

Die Verbindungen der Formel X sind neu. Bevorzugt seien die Verbindungen der Formel X genannt, in denen $R^1$, $R^2$, $R^3$ bei den Verbindungen der Formel I genannten bevorzugten Bedeutungen haben.

Im einzelnen seien folgende Verbindungen der Formel X genannt:

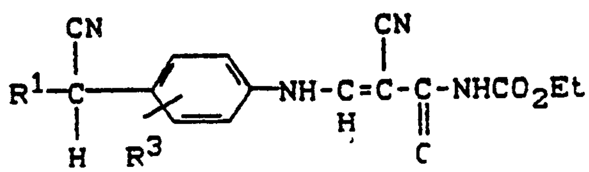

| $R^1$ | $R^3$ |
|---|---|
| 4-Cl-Phenyl | 3,5-$Cl_2$ |
| 4-Cl-Phenyl | 3-Cl |
| 4-FCS-Phenyl | 3,5-$Cl_2$ |
| 4-$F_3CSO_2$-Phenyl | 3,5-$Cl_2$ |
| 4-$OCF_3$-Phenyl | 3,5-$Cl_2$ |
| 4-$CF_3$-Phenyl | 3,5-$Cl_2$ |
| 2-Pyridyl | 3,5-Cl |
| 3,4-Cl-Phenyl | 3,5-Cl |
| 3,4-Cl-Phenyl | 3,5-Cl |
| 4-$CF_3$,3-Cl-Phenyl | 3-Cl |
| 4-$SCF_3$-Phenyl | 3-Cl |
| 4-$CH_3$-Phenyl | 3-Cl |
| 4-$OCF_3$-Phenyl | 3-Cl |
| 2-Benzthiazolyl | 3,5-Cl |
| 4-$SO_2CH_3$-Phenyl | 3,5-Cl |
| 4-F-Phenyl | 3,5-Cl |

Das Verfahren wird durchgeführt, indem man eine Verbindung der Formel (X) erhitzt, gegebenenfalls in Gegenwart eines Lösungsmittels und einer Base.

Als Lösungsmittel und Basen finden die bei der Herstellung der Verbindungen (I) aufgeführten Lösungsmittel Verwendung. Als besonders bevorzugte organische Lösungsmittel werden Alkohole wie z.B. Ethanol oder organische Säuren wie z.B. Eisessig eingesetzt.

Besonders bevorzugte Basen sind die Hydroxide und Acetate der Alkali- oder Erdalkalimetalle wie z.B. NaOH oder Natrium und Kaliumacetat.

Die Umsetzung erfolgt unter Normaldruck bei Temperaturen zwischen 70° C und 150° C, vorzugsweise zwischen 70° C und 100° C.

Die verwendete Base wird in 10 bis 80 %igem molarem Überschuß eingesetzt. Das Reaktionsgemisch wird nach abgeschlossener Cyclisierung vorzugsweise mit einer verdünnten Mineralsäure wie z.B. Salzsäure angesäuert und das als Feststoff anfallende Produkt abfiltriert.

Wird im Verfahren 10) zur Herstellung der Verbindungen (X) 2,6-Dichlor-α-(4′-methylphenyl)-4-aminophenyl-acetonitril und als Verbindung der Formel (XI) N-(2-Cyano-3-ethoxyacryloyl)urethan eingesetzt, läßt sich das Verfahren durch folgendes Schema beschreiben:

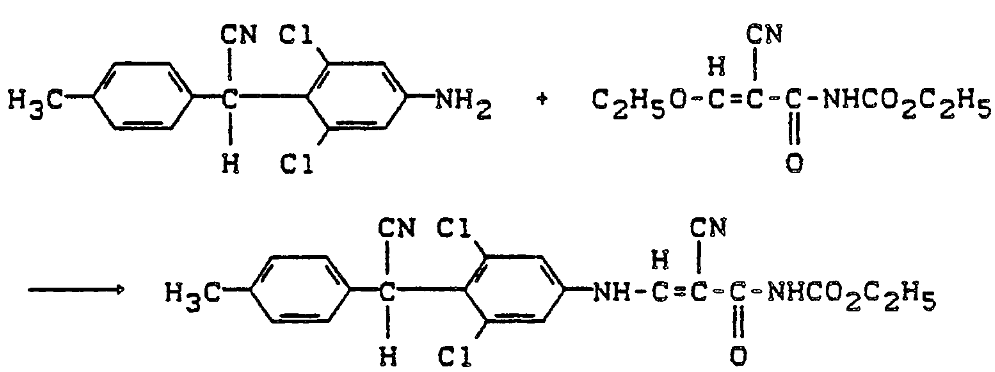

Das Verfahren wird durchgeführt, indem man Verbindungen der Formel (V) mit etwa äquimolarer Menge N-(2-Cyano-3-alkoxy-acryloyl)urethan in einem Verdünnungsmittel erhitzt und den nach Abkühlen angefallenen Feststoff absaugt.

Als Lösungsmittel kommen die bei Verfahren 2a) genannten in Frage.

Die Reaktion wird zwischen 50°C und 150°C, bevorzugt zwischen 80°C und 100°C, durchgeführt.

Die Wirkstoffe eignen sich bei günstiger Warmblütertoxizität zur Bekämpfung von parasitischen Protozoen die und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. dabliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I.rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Darüber hinaus sind die erfindungsgemäßen Verbindungen wirksam gegenüber verschiedenen zu den Helminthen (Würmern) zählenden Fischparasiten.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehoren Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens,

Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen

Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:
Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesodnere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Zu den Parasiten bei Fischen gehören aus dem Unterreich der Protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Pleistophora spp., aus dem Stamm der Plathelminthen: Trematoden; Monogenea z.B. Dactylogyrus spp., Gyrodactylus spp., Pseudodactylogyrus spp., Diplozoon spp., Cestoden, z.B. aus den Gruppen der Caryphyllidea (z.B. Caryophyllaeus laticeps), Pseudophyllidea (z.B. Diphyllobothrium spp.), Tetraphyllidea (z.B. Phyllobothrium spp.) und Protocephalida (z.B. Arten der Gattung Proteocephalus) und aus dem Stamm der Arthropoda verschiedene parasitische Crustaceen, insbesondere aus den Unterklassen der Branchiura (Fischläuse) und Copepoda (Ruderfußkrebse) sowie den Ordnungen der Isopoda (Arseln) und Amphipoda (Flohkrebse).

Die Behandlung der Fische erfolgt entweder oral, z. B. über das Futter oder durch Kurzzeitbehandlung, "medizinisches Bad", in das die Fische eingesetzt und in dem sie eine Zeitlang (Minuten bis mehrere Stunden) z. B. beim Umsetzen von einem Zuchtbecken zum anderen gehalten werden.

Es kann aber auch eine vorübergehende oder dauernde Behandlung des Lebensraums der Fische (z. B. ganzer Teichanlagen, Aquarien, Tanks oder Becken), in denen die Fische gehalten werden, erfolgen.

Der Wirkstoff wird in Zubereitungen verabreicht, die den Anwendungen angepaßt sind.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teichbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlösliche Substanz zugefügt

wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7-10, vorzugsweise aber einen pH-Wert von 8-10 haben.

Die Konzentration des Wirkstoffes kann im Bereich von 0,5-50 % liegen, vorzugsweise aber in einem Bereich von 1-25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykole, Poly(oxoethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D,L-Arginin, L- bzw. D, L-Lysin, Methylglucosamin, Glucosamin, 2-Amino-2-hydroxymethylpropandiol-(1,3) ferner wie N,N,N′,N′-tetrakis-(2-hydroxypropyl)-ethylendiamin oder Polyether-Tetrol auf der Basis Ethylendiamin (M.G. 480-420), anorganische Basen, wie Ammoniak oder Natriumcarbonat-gegebenenfalls unter Zugabe von Wasser.

Die Zubereitungen können auch 0,1 bis 20 Gew.-%, vorzugsweise 0,1-10 Gew.-% anderer Formulierhilfsstoffe, wie Antioxydantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2-50 mg Wirkstoff pro Liter Wasser bevorzugt 5-10 mg pro Liter, bei einer Behandlungsdauer von 3-4 Stunden. Bei der Behandlung von jungen Karpfen wird z.B. mit einer Konzentration von 5-10 mg/l und einer Behandlungsdauer von ca. 1-4 Stunden gearbeitet.

Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1-5 mg Wirkstoff pro Liter Wasser verwendet werden.

Zubereitungen zur Anwendung als Futterzusatz sind z.B. wie folgt zusammengesetzt:

| | |
|---|---|
| a) Wirkstoff der Formel I | 1 - 10 Gewichtsteile |
| Sojabohnen-Protein | 49 - 90 Gewichtsteile |

| | |
|---|---|
| b) Wirkstoff der Formel I | 0,5 - 10 Gewichtsteile |
| Benzylalkohol | 0,08 - 1,4 Gewichtsteile |
| Hydroxypropylmethylcellulose | 0 - 3,5 Gewichtsteile |
| Wasser | Rest ad 100 |

Zubereitungen zur Anwendung bei "medizinischen Bädern" und zur Teichbehandlung sind z.B. wie folgt zusammengesetzt und hergestellt.

c) 2,5 g Wirkstoff der Formel (I) werden in 100 ml Triethanolamin unter Erwärmen gelöst.

d) 2,5 g Wirkstoff der Formel (I)

12,5 g Milchsäure werden in 100 ml Triethanolamin unter Erwärmen und Rühren gelöst.

e) 10,0 g Wirkstoff der Formel (I) wird in 100 ml Monoethanolamin gelöst.

| | |
|---|---|
| f) Wirkstoff der Formel I | 5,0 g |
| Propylenglykol | 50,0 g |
| Natriumcarbonat | 5,0 g |
| Wasser | ad 100 ml |

| | |
|---|---|
| g) Wirkstoff der Formel I | 5,0 g |
| Monoethanolamin | 10 g |
| N-Methylpyrrolidon | ad 100 ml |

| | |
|---|---|
| h) Wirkstoff der Formel I | 2,5 g |
| Natriumcarbonat | 5,0 g |
| Polyethylenglykol200 | ad 100 ml |

Der Wirkstoff wird unter Erwärmen im Polyethylenglykol gelöst und Natriumcarbonat darin suspendiert.

## Beispiel A

Coccidiose bei Hühnern

9 bis 11 Tage alte Küken wurden mit 40000 sporulierten Oozysten von stark virulenten Stämmen von Eiveria acervulina, E. maxima und E. tenella, den Krankheiteserregern der intestinalen Coccidiose infiziert.

3 Tage vor der Infektion und 8 Tage nach der Infektion (Ende des Versuchs) wurde Wirkstoff in der angegebenen Konzentration im Futter der Tiere eingemischt verabreicht.

Die Zahl der Oozysten im Kot wurde mit Hilfe der McMaster-Kammer bestimmt (siehe Engelbrecht und Mitarbeiter "Parasitologische Arbeitsmehoden in Medizin und Veterinärmedizin", S. 172, Akademie-Verlag, Berlin (1965)).

Als wirksam werden diejenigen Dosen angesehen, die die Ausscheidung von Oozysten und/oder klinische Symptome der Coccidiose einschließlich der Mortalität vollständig oder in hohem Maße verhüten. In der folgenden Tabelle werden die wirksamen Dosen angegeben:

Tabelle 1

| Coccidiose bei Hühnern | | | | | |
|---|---|---|---|---|---|
| Beispiel Nr. | Dosis ppm. | Sterberate tot/eingesetzt | Oocystenausscheidung in % im Vergleich zur unbehandelten infizierten Kontrolle | Gewichtszunahme in % im Vergleich zur nicht infizierten unbehandelten Kon-Kontrolle | Blutausscheidung mit dem Kot |
| unbehandelte infizierte Kontrolle | | 2/6 | 100 | 35 | stark |
| 1 | 50 | 0/3 | 0 | 100 | keine |

Herstellungsbeispiele

I Beispiele für Verfahren 2a

Beispiel 1

2,6-Dichloro-α-(4-chlorphenyl)-4-(1-uracil)-phenylacetonitril

7,45 g (0,018 Mol) 2,6-Dichlor-α-(4′-chlorphenyl)-4-(3-ethoxy-acryloyl)ureido-phenylacetonitril werden in 150 ml tert. Butanol gelöst und mit 2,12 g (0,018 Mol) Kalium tert. Butylat versetzt und 10 Minuten zum Sieden erhitzt. Das Lösungsmittel wird im Vakuum abdestilliert und der Rückstand im Vakuum auf 100 °C für 30 Minuten erhitzt. Anschließend verrührt man mit Wasser, säuert mit Essigsäure an, filtriert den ausgefallenen Feststoff ab und kristallisiert aus Ethanol um. Man erhält so 6,2 g (78 % der Theorie) 2,6-Dichlor-α-(4-chlorphenyl)-4-(1-uracil)-phenylacetonitril.

Analog werden hergestellt:

Beispiel 2

2,6-Dichlor-α-(4-trifluormethylthio)-4-(1-uracil)-phenylacetonitril

Beispiel 3

2,6-Dichlor-α-(4′-trifluormethoxy)-4-(1-uracil)-phenylacetonitril

Beispiel 4

2,6-Dichlor-α-(4-trifluormethylsulfonyl)-4-(1-uracil)-phenylacetonitril

Beispiel 5

2,6-Dichlor-α-(2-benzthiazolyl)-4-(1-uracil)-phenylacetonitril

II Beispiel für Verfahren 2b

Beispiel 6

2,6-Dichlor-α-(2′,4′-dichlorphenyl)-α-methyl-4-(3-N-methyl-1-uracil)-phenylacetonitril

4,5 g (0,01 Mol) 2,6-Dichlor-α-(2′,4′-dichlorphenyl)-α-methyl-4-(1-uracil)-phenylacetonitril werden in 50 ml absolutem DMSO gelöst und mit 0,23 g (10 mMol) NaH versetzt. Man rührt 20 Minuten bei Raumtemperatur und gibt dann 1,85 g (0,013 Mol) Methyliodid in 5 ml DMSO zu. Man erwärmt auf 50 °C und hält 6

Stunden bei dieser Temperatur, engt dann im Vakuum ein und versetzt den Rückstand mit Wasser. Nach dem Absaugen des ausgefallenen Feststoffs erhält man so 2,64 g (57 % der Theorie) der N-Methylverbindung.

Beispiele für Verfahren 2c

Beispiel 7

2,6-Dichlor-α-(2′,6′-dichlorphenyl)-4-(1-uracil)-phenylacetonitril

5,6 g (0,012 Mol) 2,6-Dichlor-α-(2′,6′-dichlorphenyl)-4-(5′-carboxy-1′-uracil)-phenylacetonitril werden in 10 ml Mercaptoessigsäure auf 170° C erhitzt. Nach 1 Stunde läßt man abkühlen, versetzt mit Wasser und erhält nach dem Absaugen des Niederschlags 3,4 g (67 % der Theorie) 2,6-Dichlor-α-(2′,6′-dichlorphenyl)-4-(1′-uracil)-phenylacetonitril.

Beispiel 8

2,6-Dichlor-α-(4′-methylphenyl)-4-(1-uracil)-phenylacetonitril

Beispiel 9

2,6-Dichlor-α-(2′-pyridinyl)-4-(1-uracil)-phenylacetonitril

Beispiel 10

2,6-Dichlor-α-(4′-trifluormethylphenyl)-4-(1-uracil)-phenylacetonitril

Beispiel für Verfahren 4

Beispiel 11

2,6-Dichlor-α-(4′-chlorphenyl)-4-N(3-ethoxyacryloyl)-ureido-phenylacetonitril

10,7 g (0,034 Mol) 2,6-Dichloro-α-(4′-chlorphenyl)-4-aminophenylacetonitril werden in 70 ml abs. Toluol gelöst. Hierzu tropft man 150 ml einer aus 8,5 g Silbercyanat und 5,6 g Ethoxyacrylsäurechlorid bereitete toluolische Lösung und rührt 1 Stunde bei 40° C nach. Der ausgefallene Feststoff wird abgesaugt mit Petrolether gewaschen und getrocknet. Man erhält so 12,6 g (82 % der Theorie) 2,6-Dichloro-α-(4′-chlorphenyl)-4-(3-ethoxyacryloyl)ureido-phenylacetonitril.

Analog werden hergestellt:

Beispiel 12

2,6-Dichlor-α-(3′,4′-dichlorophenyl)-4-N(3-ethoxyacryloyl)-ureido-phenylacetonitril

Beispiel 13

2,6-Dichlor-α-(4′-trifluormethoxyphenyl)-4-N(3-ethoxyacryloyl)-ureido-phenylacetonitril

Beispiel 14

2,6-Dichlor-α-(4′-trifluormethylthiophenyl)-4-N(3-ethoxyacryloyl)-ureido-phenylacetonitril

Beispiel für Verfahren 4b

Beispiel 15

2,6-Dichlor-α-(4′-chlorphenyl)-4-N(3-ethoxyacryloyl)-N′-methylureido-phenylacetonitrl

8,6 g (0,025 mol) 4-Isocyanato-2,5-dichlor-α-(4-chlorphenyl)-α-methyl-phenylacetonitril werden in absolutem Tetrahydrofuran gelöst. Hierzu tropft man 3,2 g (0,025 mol) Ethoxyacryloyl-N-methylamid und gibt dann portionsweise 600 mg (0,025 mol) Natriumhydrid zu. Man erwärmt 1 h auf 40° C und dann weitere 3 h auf60° C. Nach dem Abkühlen suagt man den ausgefallenen Feststoff ab und kristallisiert aus Ethanol um. Man erhält so 6.2 g (52 % d.Th.) 2,6-Dichlor-α-(4′-chlorphenyl)-4-N-(3-ethoxyacryloyl)-N′-methylureido-phenylacetonitril.

Beispiel für Verfahren 6

Beispiel 16

2,6-Dichlor-α-(4′-methylphenyl)-4-(5-carboxy-1-uracil)-phenylacetonitril

13,5 g (0,033 Mol) 2,6-Dichlor-α-(4′-methylphenyl)-4-(5-carboxy-1-uracil)phenylacetonitril werden 200 ml HCl/Eisessig (1:1) unter Rückfluß gerührt. Anschließend versetzt man mit Wasser und saugt die ausgefallene Carbonsäure ab, 8,9 g (63 % der Theorie).

Analog werden hergestellt:

Beispiel 17

2-Chlor-α-(4′-chlorphenyl)-4-(5-carboxy-1-uracil)-phenylacetonitril

Beispiel 18

2,6-Dichlor-α-phenyl-4-(5-carboxy-1-uracil)phenylacetonitril

Beispiel 19

2,6-Dichlor-α-(4′-methylphenyl)-4-(1-uracil)-phenylacetonitril

12 g (0,03 Mol) Cyanuracil werden in 50 ml HCl konz. und 30 ml Mercaptoessigsäure 36 Stunden unter Rückfluß gerührt. Nach dem Abkühlen verdünnt man mit Wasser und saugt den ausgefallenen Niederschlag ab. Umkristallisation aus Ethanol liefert 7,9 g (70 % der Theorie) 2,6-Dichlor-α-(4′-methylphenyl)-4-(1-uracil)-phenylacetonitril.

Analog werden hergestellt:

Beispiel 20

2,6-Dichlor-α-(2′-chlorphenyl)-4-(1-uracil)-phenylacetonitril

Beispiel 21

2,6-Dichlor-α-(3′-methylphenyl)-4-(1-uracil)-phenylacetonitril

Beispiel 22

2,6-Dichlor-α-(2′-fluor-6′-chlorphenyl)-4-(1-uracil)-phenyl-acetonitril

Beispiel für Verfahren 8

Beispiel 23

2,6-Dichlor-α-(4′-methylphenyl)-4-(5-cyano-1-uracil)-phenylacetonitril

17,2 g (0,038 Mol) 2,6-Dichloro-α-(4′-methylphenyl)-4-[N-(2-cyanoacryloylurethan]amino-phenylacetonitril werden mit 4,2 g (0,05 Mol) Natriumacetat in 200 ml Ethanol 2 Stunden unter Rückfluß erhitzt. Nach dem Abkühlen säuert man mit 2 n HCl an und saugt den ausgefallenen Niederschlag ab. Man erhält so 12,2 g (74 % der Theorie) 2,6-Dichlor-α-(4′-methylphenyl)-4-(5-cyano-1-uracil)-phenylacetonitril.

Analog werden hergestellt:

Beispiel 24

2,6-Dichlor-α-(4-chlorphenyl)-4-(5-cyano-1-uracil)-phenylacetonitril

Beispiel 25

2,6-Dichlor-α-(4-trifluorphenyl)-4-(5-cyano-1-uracil)-phenylacetonitril

Beispiel für Verfahren 10

Beispiel 26

2,6-Dichlor-α-(4-methylphenyl)-4-N-(2-cyanoacryloylurethan)amino-phenylacetonitril

25 g (0,086 Mol) 2,6-Dichlor-α-(4′-methylphenyl)-4-amino-phenylacetonitril werden mit 17,8 g (0,09 Mol) N-(2-Cyano-3-ethoxyacryloyl)urethan in 200 ml Ethanol 4 Stunden unter Rückfluß gerührt. Anschließend engt man auf die Hälfte ein und läßt das Produkt auskristallisieren. Man erhält so 28,2 g (72 % der Theorie) 2,6-Dichlor-α-(4-methylphenyl)-4-N-(2-cyano-acryloylurethan)amino-phenylacetonitril.

Analog werden hergestellt:

Beispiel 27

2,6-Dichlor-α-(4′-chlorphenyl)-4-N-(2-cyanoacryloylurethan)amino-phenylacetonitril

Beispiel 28

2,6-Dichlor-α-methyl-α-phenyl-4-N-(2-cyano-acryloylurethan)-amin phenylacetonitril

**Ansprüche**

1. Neue substituierte Uracile der allgemeinen Formel (I)

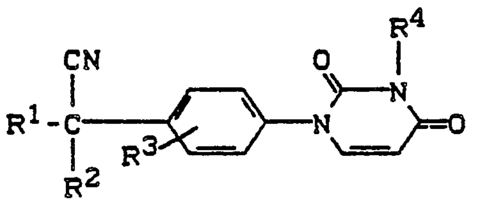

(I)

in welcher

$R^1$ für aromatische oder heteroaromatische Reste steht, die gegebenenfalls substituiert sind,

$R^2$ für H, Alkyl, Alkenyl, Alkinyl oder Aralkyl steht, die gegebenenfalls substituiert sind,

$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl steht,

$R^4$ für Wasserstoff, einen geradkettigen, verzweigten oder cyclischen Alkylrest, Alkenyl, Alkinyl oder Aralkyl steht, die gegebenenfalls substituiert sind.

2. Verfahren zur Herstellung substituierter Uracile der allgemeinen Formel (Ia)

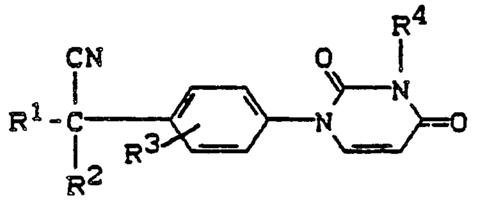

(I)

in welcher

$R^1$ für aromatische oder heteroaromatische Reste steht, die gegebenenfalls substituiert sind,

$R^2$ für Wasserstoff, Alkyl, Alkenyl, Alkinyl oder Aralkyl steht, die gegebenenfalls substituiert sind,

$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl steht,

$R^4$ für Wasserstoff, einen geradkettigen, verzweigten oder cyclischen Alkylrest, Alkenyl, Alkinyl oder Aralkyl steht, die gegebenenfalls substituiert sind,
indem man
a) Verbindungen der Formel (II)

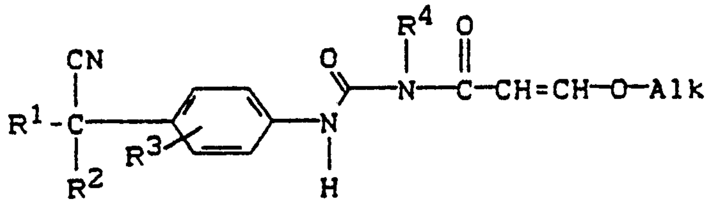

(II)

in welcher
$R^1$, $R^2$, $R^3$, $R^4$ die oben angegebenen Bedeutungen haben und
Alk für $C_1$-$C_4$-Alkyl insbesondere Ethyl steht,
gegebenenfalls in Gegenwart von Basen erhitzt, oder indem man
b) Verbindungen der Formel (Ia)

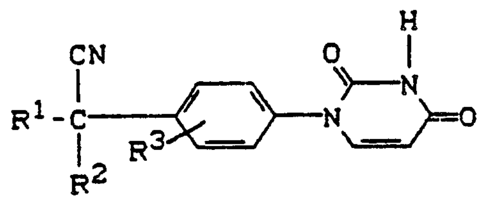

(Ia)

in welcher $R^1$, $R^2$, $R^3$ die oben angegebenen Bedeutungen haben,
mit Verbindungen der Formel (III)
$R^4$ - A
in welcher
$R^4$ für gegebenenfalls substituiertes Alkyl, Alkenyl, Alkinyl oder Aralkyl steht und
A für Halogen, $-OSO_2$-Alkyl, $-OSO_2$-Aryl, $-OSO_2$-Halogenalkyl steht,
oder indem man
c) Verbindungen der Formel (IV)

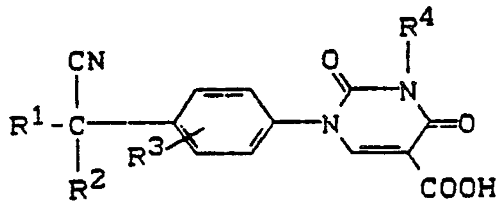

(IV)

in welcher
$R^1$, $R^2$, $R^3$, $R^4$ die oben angegebenen Bedeutungen haben,
durch Erhitzen decarboxyliert.

3. Neue Verbindungen der Formel (II)

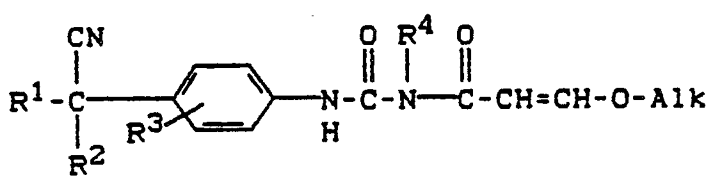

(II)

in welcher
$R^1$, $R^2$, $R^3$, $R^4$, Alk die in Anspruch 2) angegebenen Bedeutungen haben.

4. Verfahren zur Herstellung der Verbindungen der Formel (II) gemäß Anspruch 3, dadurch gekennzeichnet, daß man

a) Verbindungen der Formel (V)

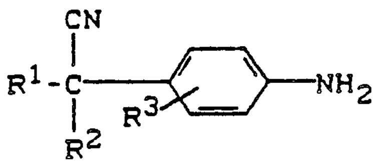

(V)

in welcher

$R^1$, $R^2$, $R^3$ die obengenannten Bedeutungen besitzen,

mit Isocyanaten der Formel (VI)

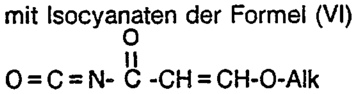

(VI)

in welcher

Alk die obengenannte Bedeutung besitzt,

umsetzt, oder

b) Verbindungen der Formel (VII)

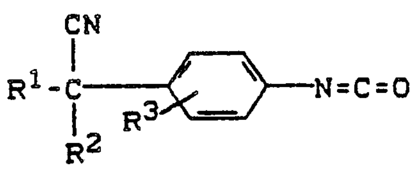

(VII)

in welcher $R^1$, $R^2$, $R^3$ die obengenannten Bedeutungen besitzen,

mit Verbindungen der Formel (VIII)

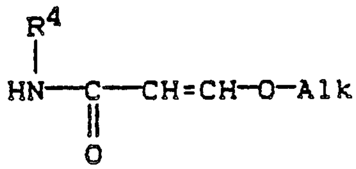

(VIII)

in welcher

$R^4$ oder Alk die obengenannten Bedeutungen besitzt, umsetzt.

5. Neue Verbindungen der Formel (IV)

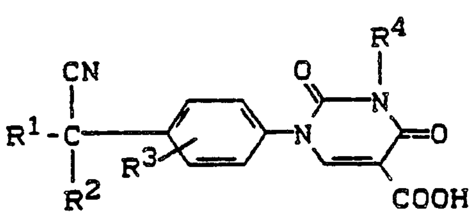

(IV)

in welcher

$R^1$, $R^2$, $R^3$, $R^4$ die in Anspruch 1 angegebenen Bedeutungen besitzen.

6. Verfahren zur Herstellung der neuen Verbindungen der Formel (IV) gemäß Anspruch 5, dadurch gekennzeichnet, daß man Verbindungen der Formel (IX)

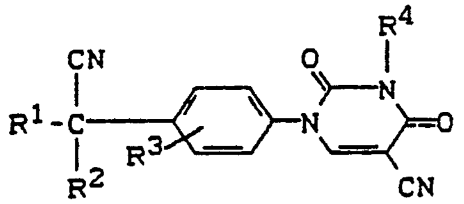

(IX)

in welcher
$R^1$, $R^2$, $R^3$, $R^4$ die in Anspruch 5 angegebenen Bedeutungen besitzen,
in Gegenwart wäßriger Säuren erhitzt.

7. Neue Verbindungen der Formel (IX)

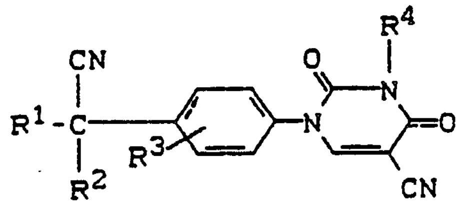

(IX)

in welcher
$R^1$, $R^2$, $R^3$, $R^4$ die in Anspruch 5 angegebenen Bedeutungen haben.

8. Verfahren zur Herstellung der neuen Verbindungen der Formel (IX) gemäß Anspruch 7, dadurch gekennzeichnet, daß man Verbindungen der Formel (X)

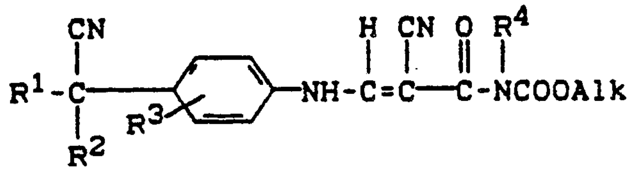

(X)

in welcher
$R^1$, $R^2$, $R^3$, $R^4$, Alk die in Anspruch 7 angegebene Bedeutung besitzen,
in Gegenwart von Basen erhitzt.

9. Neue Verbindungen der Formel (X)

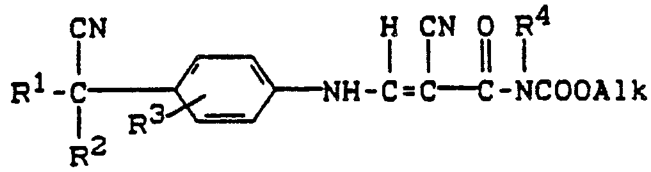

(X)

in welcher
$R^1$, $R^2$, $R^3$, $R^4$ und Alk die in Anspruch 4 b) beschriebenen Bedeutungen besitzen.

10. Verfahren zur Herstellung der neuen Verbindungen der Formel (X) gemäß Anspruch 9, dadurch gekennzeichnet, daß man Verbindungen der Formel (V)

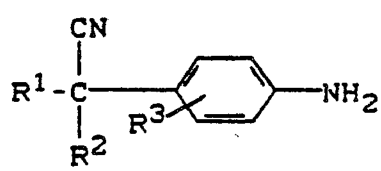

(V)

in welcher

$R^1$, $R^2$, $R^3$ die in Anspruch 4 beschriebenen Bedeutungen besitzen
mit Verbindungen der Formel (XI)

```
                CN  O  R4
                |   ||  |
Alk-O-CH=C——C-N-COOAlk          (XI)
```

in welcher
Alk für gleiches oder verschiedenes $C_1$-$C_4$-Alky steht und $R^4$ die in Anspruch 4b beschriebene Bedeutung besitzt,
umsetzt.

11. Mittel gegen parasitäre Protozoen, gekennzeichnet durch einen Gehalt an mindestens einem substituierten Uracil der Formel (I) gemäß Anspruch 1.

12. Verwendung von substituierten Uracilen der Formel (I) gemäß Anspruch 1 zur Bekämpfung von parasitären Protozoen.

13. Verfahren zur Bekämpfung von parasitären Protozoen, dadurch gekennzeichnet, daß man substituierte Uracile der Formel (I) gemäß Anspruch 1 auf diese und/oder ihren Lebensraum einwirken läßt.

14. Verfahren zur Herstellung von Mitteln gegen parasitäre Protozoen, dadurch gekennzeichnet, daß man substituierte Uracile der Formel (I) gemäß Anspruch 1 mit Streckmitteln und/oder oberflächenaktiven Mitteln vermischt.

15. Verwendung von substituierten Uracile der Formel (I) gemäß Anspruch 1 zur Herstellung von Mitteln gegen parasitäre Protozoen.